# EUROPEAN PATENT APPLICATION

(11) **EP 4 729 093 A1**
(43) Date of publication of application: **22.04.2026**
(21) Application number: 24826063.0
(22) Date of filing: 06.03.2024
(51) Int. Cl.: A61M 5/30, A61M 5/315, A61M 39/24, A61M 5/31

(54) **LIQUID JET INJECTION DEVICE**

(30) Priority: 19.06.2023 KR 20230078166
(71) Applicant: Kaosys Corp., Cheonan-si, Chungcheongnam-do 31035 (KR)
(72) Inventor: LEE, Soo Yeun, Pohang-si Gyeongsangbuk-do 37800 (KR); BANG, Jin SU, Pohang-si Gyeongsangbuk-do 37800 (KR)
(74) Representative: AWA Sweden AB
(86) International application number: PCT/KR2024/002864
(87) International publication number: WO 2024/262738

(57) **Abstract**

Disclosed is a liquid jet injection device for injecting a liquid medicine into the tissue of an organism without an injection needle. The liquid jet injection device comprises: a cylinder; a casing that surrounds the cylinder; a cylinder part that is accommodated in the cylinder and provided with a hammer that can move between one end and the other end of the cylinder; an intake flow path that connects one end of the cylinder and a compressed air-generating part that generates compressed air that is supplied to the cylinder; a control valve for selectively opening and closing an exhaust flow path that connects the one end of the cylinder to the outside; a pressurizing part provided with a piston that faces an opening formed at the other end of the cylinder; and a nozzle part that is coupled to the pressurizing part, wherein the nozzle part discharges the liquid stored therein to the outside when the hammer collides with the piston. A charging part, which communicates with the other end of the cylinder and in which compressed air is compressed and charged when the intake flow path is opened, is provided between the casing and the cylinder. When the exhaust flow path is opened in a state in which the hammer has moved from the one end to the other end of the cylinder due to the opening of the intake flow path, the hammer is moved from the one end to the other end of the cylinder by the compressed air compressed and charged in the charging part.

## Description

### [Technical Field]

The present invention relates to a device capable of accelerating a liquid and injecting the liquid into a tissue such as skin and the like desired by a user.

### [Background Art]

Generally, a technology of accelerating a liquid to cut or penetrate a target body has been widely used. A representative example is an ultra-high-pressure cutting method called waterjet cutting. A waterjet is an apparatus that cuts the target body into a desired shape by spraying water or an abrasive mixture pressurized to ultra-high pressure onto a surface of the target body through an orifice and a nozzle.

Meanwhile, in general, in order to inject a drug into a tissue, a syringe is used. Looking at a structure of the syringe, the syringe is composed of a needle portion to be inserted into the tissue, a cylinder for charging a liquid medicine, and a piston inserted into and moved inside the cylinder. Such a syringe is configured to penetrate the tissue by moving the drug charged into the cylinder using the piston.

Although the syringe is an efficient delivery means of the drug, a possibility of infection caused by the needle, a fear of needles, and inconvenience of use have been raised, and accordingly, recently, research into a needle-free syringe has been continuously conducted.

As an example of the needle-free syringe, when an energy source capable of pressurizing a piston of the syringe cylinder is configured, and a structure of an orifice and a nozzle like the waterjet is used, it is possible to implement a drug delivery device capable of stably delivering the drug while continuously supplying the drug instead of the abrasive mixture. In this case, a high-pressure waterjet stream is mixed with a mixture supplied from the outside and then passes through the nozzle at a very high speed. By using this, a structure is possible in which the waterjet is controlled to operate only at a certain moment, and in accordance with the corresponding operation sequence, the drug is sequentially supplied from the outside.

Meanwhile, among the needle-free syringes, there are methods of injecting the drug filled in the cylinder into the skin by compressing air using strong air pressure, and applying pressure to the piston of the cylinder. In such methods, excessively large amounts of the drug are consumed, and due to a high output intensity, damage is caused to the skin tissue and so on, and a continuous injection speed is relatively low, so there are many restrictions in actual use. In order to solve such conventional problems, development of a needle-free syringe in which an operation mechanism for injecting the drug is more simply configured may be considered.

### [Disclosure]

### [Technical Problem]

It is an object of the present invention to provide a liquid jet injection device in which a configuration is more simply configured so as to be capable of stably and repeatedly operating a process of generating a waterjet caused by an operation of a piston configured to move a drug using air pressure.

### [Technical Solution]

To achieve the object of the present invention, there is provided a liquid jet injection device, according to an embodiment of the present invention. The liquid jet injection device may comprise: a cylinder part provided with a cylinder, a casing surrounding the cylinder, and a hammer accommodated in an inside of the cylinder and movably formed between one end and the other end of the cylinder; a control valve configured to selectively open and close an intake flow path connecting a compressed air-generating part generating compressed air supplied into the inside of the cylinder and one end of the cylinder, and an exhaust flow path connecting one end of the cylinder and the outside; a pressurizing part provided with a piston facing an opening formed at the other end of the cylinder; and a nozzle part coupled to the pressurizing part and formed to discharge to the outside a liquid medicine stored therein when the hammer collides with the piston, in which a charging part configured to communicate with the other end of the cylinder and in which the compressed air is compressed and charged when the intake flow path is opened, may be provided between the casing and the cylinder, and the hammer, in a state of having moved from one end of the cylinder to the other end of the cylinder by opening of the intake flow path, may be configured to move from the other end of the cylinder to one end of the cylinder by the compressed air compressed and charged in the charging part when the exhaust flow path is opened.

According to an example related to the present invention, the hammer, when the intake flow path is opened, may move to the other end of the cylinder by the compressed air supplied to one end of the cylinder and collide with the piston, and in a state in which the hammer has moved to the other end of the cylinder, when the exhaust flow path is opened, the hammer may be moved to one end of the cylinder by being pushed by an air flow in which the compressed air charged in the charging part escapes to the outside of the cylinder through the exhaust flow path due to an applied pressure difference between the inside of the cylinder and the outside.

According to an example related to the present invention, the charging part may be formed to surround a part of a side surface of the cylinder.

According to an example related to the present invention, a partition plate may be provided between the other end of the cylinder and the piston, and a communication part formed to communicate an opening formed at the other end of the cylinder with the charging part may be provided in the partition plate.

According to an example related to the present invention, a connection flow path communicating with the inside of the cylinder, the intake flow path, and the exhaust flow path may be provided between the intake flow path and the exhaust flow path, and the control valve may control the connection flow path to selectively communicate with any one of the intake flow path and the exhaust flow path.

According to an example related to the present invention, the piston may comprise: a first piston facing an opening formed at the other end of the cylinder and configured to collide with the hammer; and a second piston disposed in the nozzle part, facing the first piston, and configured to collide with the first piston, which has collided with the hammer as the intake flow path is opened, so as to discharge a liquid medicine stored inside the nozzle part to the outside.

According to an example related to the present invention, the nozzle part may comprise: a nozzle housing configured to accommodate the second piston; and a piston cushion interposed between the nozzle housing and the second piston, and configured to provide a resilient force to the second piston so as to restore the second piston moved by the collision with the first piston.

According to an example related to the present invention, a magnetic body having a magnetic force may be provided in the casing facing one end of the cylinder, and the hammer may be formed to be coupled with the magnetic body by the magnetic force.

According to an example related to the present invention, a hammer cushion formed to absorb impacts when the magnetic body and the hammer collide with each other may be provided on at least one of two surfaces facing each other between the magnetic body and the hammer.

There is provided a liquid jet injection device, according to another embodiment of the present invention. The liquid jet injection device may comprise: a cylinder part provided with a cylinder, a casing surrounding the cylinder, and a hammer accommodated in an inside of the cylinder and movably formed between one end and the other end of the cylinder; a control valve configured to selectively open and close an intake flow path connecting a compressed air-generating part generating compressed air supplied into the inside of the cylinder and one end of the cylinder, and an exhaust flow path connecting one end of the cylinder and the outside; a pressurizing part provided with a piston facing an opening formed at the other end of the cylinder; and a nozzle part coupled to the pressurizing part and formed to discharge to the outside a liquid medicine stored therein when the hammer collides with the piston, in which the hammer, when the exhaust flow path is opened, may be configured to be restored from the other end of the cylinder to one end of the cylinder by the compressed air, which is caused to be compressed inside the casing when the intake flow path is opened.

### [Advantageous Effects]

The effects of the present invention obtained by the above-described solution are as follows.

The liquid jet injection device includes a cylinder part provided with a cylinder accommodating a hammer and a casing surrounding the cylinder. Here, between the casing and the cylinder, a charging part communicating with the other end of the cylinder and charged with compressed air is provided. In the charging part, in a state in which the compressed air is compressed and charged when an intake flow path forming a flow path through which the compressed air flows into the inside of the cylinder is opened, when an exhaust flow path formed at one end of the cylinder is opened, the compressed air of the charging part moves the hammer back to an initial position by an applied pressure difference between the inside of the cylinder and the outside.

According to the configuration of the liquid jet injection device, a separate configuration is not required to move the hammer moved by the compressed air back to the initial position, and the device may be implemented by an operation of opening the exhaust flow path through a control valve. Therefore, since the configuration for the operation of the liquid jet injection device may be more simply constituted, manufacturing cost of the liquid jet injection device may be reduced to secure price competitiveness. In addition, by simplifying the components of the liquid jet injection device, a failure rate may be lowered, and maintenance cost may be reduced.

### [Description of Drawings]

FIG. 1 is a conceptual diagram of a liquid jet injection device according to an embodiment of the present invention.
FIG. 2 is a conceptual diagram illustrating a state in which a part of the liquid jet injection device illustrated in FIG. 1 is separated.
FIG. 3 is a cross-sectional view taken along line A-A illustrated in FIG. 2.
FIG. 4 is a conceptual diagram illustrating a state before an intake flow path of the liquid jet injection device illustrated in FIG. 1 is opened.
FIG. 5 is a conceptual diagram illustrating a state in which the intake flow path is opened in the liquid jet injection device illustrated in FIG. 4, and the hammer moves from one end of the cylinder toward the other end.
FIG. 6 is a conceptual diagram illustrating a state in which, in the liquid jet injection device illustrated in FIG. 5, the hammer moves to the other end of the cylinder, collides with the piston, and the piston moves a predetermined distance by the impact.
FIG. 7 is a conceptual diagram illustrating a state in which, in the liquid jet injection device illustrated in FIG. 6, after the hammer collides with the piston, the exhaust flow path is opened, and the hammer moves toward one end of the cylinder.
FIG. 8 is a conceptual diagram illustrating a state in which, in the liquid jet injection device illustrated in FIG. 7, the hammer moves to one end of the cylinder and returns to an initial position.

### [Mode for Disclosure]

Hereinafter, a liquid jet injection device 100 related to the present invention will be described in more detail with reference to the drawings.

In this specification, even in different embodiments, the same or similar reference numerals are assigned to the same or similar configurations, and duplicate descriptions thereof will be omitted.

An expression in singular form includes plural forms unless the context clearly indicates otherwise.

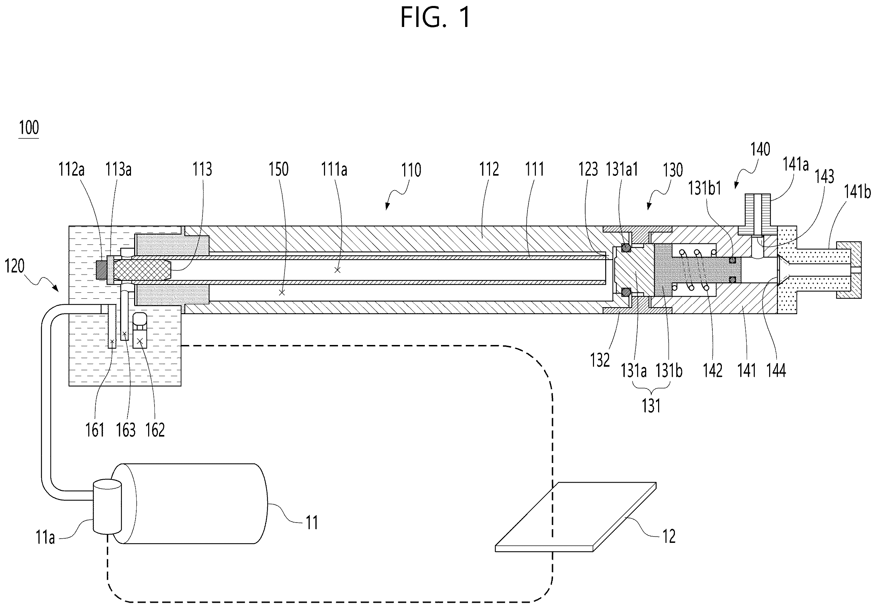

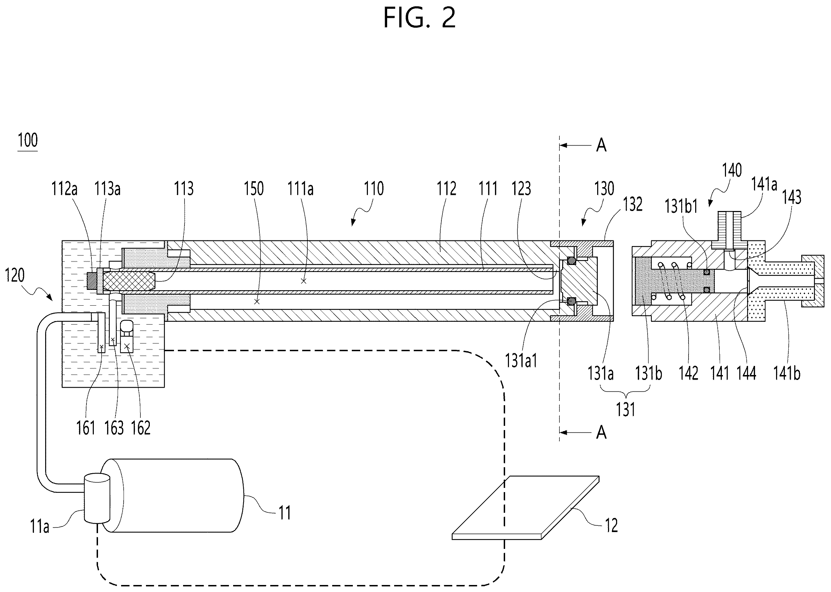

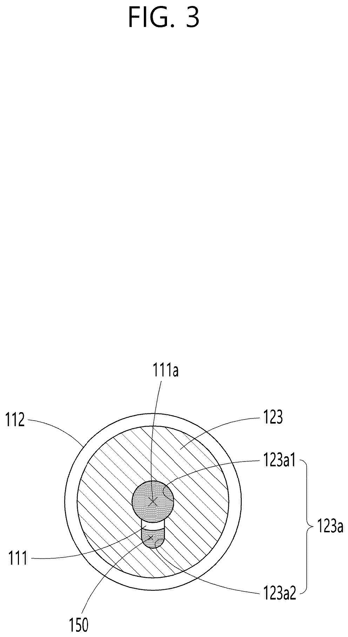

FIG. 1 is a conceptual diagram of a liquid jet injection device 100 according to an embodiment of the present invention. FIG. 2 is a conceptual diagram illustrating a state in which a part of the liquid jet injection device 100 illustrated in FIG. 1 is separated. FIG. 3 is a cross-sectional view taken along line A-A illustrated in FIG. 2.

With reference to FIGS. 1 to 3, the liquid jet injection device 100 includes a cylinder part 110, a control valve 120, a pressurizing part 130, and a nozzle part 140.

The cylinder part 110 is provided with a cylinder 111, a casing 112, and a hammer 113.

The cylinder 111 is formed to extend in one direction. Inside the cylinder 111, a space in which the hammer 113 is movably formed between one end and the other end of the cylinder 111 is provided. The cylinder 111 may be formed in a cylindrical shape, and both ends may be formed to be opened, or only the other end may be formed to be opened. In addition, the shape of the cylinder 111 is not limited to a cylindrical shape, but may be formed in another non-cylindrical polygonal column shape in which a space for movement of the hammer 113 is provided at an inside.

The casing 112 is disposed outside the cylinder 111 and is formed to surround the cylinder 111.

The hammer 113 is accommodated in an inside 111a of the cylinder 111 and is movably formed between one end and the other end of the cylinder 111. The hammer 113 may be formed corresponding to an inner surface of the cylinder 111. For example, when the shape of the cylinder 111 is cylindrical, the shape of the hammer 113 may also be formed as a cylinder or cylindrical shape. Surfaces of the cylinder 111 and the hammer 113 facing each other are formed to correspond to each other, so that the hammer 113 may move while being stably guided inside the cylinder 111.

The control valve 120 may be configured to selectively open and close an intake flow path 161 and an exhaust flow path 162.

The intake flow path 161 forms a flow path through which compressed air flows into the inside of the cylinder 111. The intake flow path 161 is configured to connect a compressed air-generating part 11 generating the compressed air and one end of the cylinder 111. That is, one side of the intake flow path 161 communicates with the compressed air-generating part 11, and the other side is formed to communicate with one end of the cylinder 111. The compressed air-generating part 11 may be provided with a pressure valve 11a for adjusting a pressure of the compressed air supplied into the inside 111a of the cylinder 111.

The exhaust flow path 162 forms a flow path connecting one end of the cylinder 111 and the outside of the cylinder 111. The outside of the cylinder 111 may be an atmospheric region.

Between the intake flow path 161 and the exhaust flow path 162, a connection flow path 163 may be provided. The connection flow path 163 communicates with the inside 111a of the cylinder 111 and the intake flow path 161, and communicates with the inside 111a of the cylinder 111 and the exhaust flow path 162.

The control valve 120 may control the connection flow path 163 to selectively communicate with any one of the intake flow path 161 and the exhaust flow path 162. That is, when the control valve 120 allows the connection flow path 163 to communicate with the intake flow path 161, the connection flow path 163 may be configured to close communication with the exhaust flow path 162. Conversely, when the control valve 120 allows the connection flow path 163 to communicate with the exhaust flow path 162, the connection flow path 163 may be configured to close communication with the intake flow path 161.

The control valve 120 may perform open and close operations of the intake flow path 161 and the exhaust flow path 162 according to whether power is applied. For example, when power is applied, the control valve 120 may be configured to connect the intake flow path 161 and the connection flow path 163, and when power is cut off, to connect the exhaust flow path 162 and the connection flow path 163.

The pressurizing part 130 is provided with a piston 131 facing an opening formed at the other end of the cylinder 111. The pressurizing part 130 may be provided with a pressurizing part cover 132 surrounding at least a part of the piston 131.

The nozzle part 140 is coupled to the pressurizing part 130 and is formed to discharge to the outside a liquid medicine stored inside when the hammer 113 collides with the piston 131.

The cylinder part 110, the pressurizing part 130, and the nozzle part 140 may be formed to be mutually separable and may be provided as a disposable structure for one-time treatment for diversification of injection techniques and prevention of infection.

Meanwhile, a charging part 150 is provided between the casing 112 and the cylinder 111.

The charging part 150 communicates with the other end of the cylinder 111 and forms a space in which compressed air is compressed and charged when the intake flow path 161 is opened.

In addition, the charging part 150 may be formed to surround a part of a side surface of the cylinder 111. For example, the charging part 150 may be formed only at a lower portion except for an upper portion of the cylinder 111 on the basis of the liquid jet injection device 100 illustrated in FIG. 1. Accordingly, since a shape and/or disposition of the charging part 150 may be more variously applied, a design of the liquid jet injection device 100 may be implemented in more diverse forms. However, the charging part 150 may be formed to surround the entirety of a side surface of the cylinder 111, not only a part thereof.

Here, when the exhaust flow path 162 is opened, the hammer 113 may be restored from the other end of the cylinder 111 to one end of the cylinder 111 by the compressed air, which is caused to be compressed inside the casing 112 when the intake flow path 161 is opened.

More specifically, in a state in which the hammer 113 has moved from one end of the cylinder 111 to the other end of the cylinder 111 by opening of the intake flow path 161, when the exhaust flow path 162 is opened, the hammer 113 is moved from the other end of the cylinder 111 to one end of the cylinder 111 by the compressed air compressed and charged in the charging part 150.

For example, when the intake flow path 161 is opened, the hammer 113 is moved to the other end of the cylinder 111 by the compressed air supplied to one end of the cylinder 111, and collides with the piston 131.

Further, in a state of having moved to the other end of the cylinder 111, when the exhaust flow path 162 is opened, the hammer 113 is configured to be moved to one end of the cylinder by being pushed by an air flow in which the compressed air compressed and charged in the charging part escapes to the outside of the cylinder 111 through the exhaust flow path 162 due to an applied pressure difference between the inside 111a of the cylinder 111 and the outside.

Meanwhile, the liquid jet injection device 100 may include a control unit 12 performing functions related to control, such as the control valve 120, the compressed air-generating part 11, and the like. The control unit 12 may include a display module (not illustrated) for a user interface.

Meanwhile, as shown in FIG. 3, a partition plate 123 may be provided between the other end of the cylinder 111 and the piston 131.

A communication part 123a may be provided in the partition plate 123.

The communication part 123a is formed to communicate an opening formed at the other end of the cylinder 111 with the charging part 150.

In addition, the communication part 123a of the partition plate 123 may be provided with a first hole 123a1 communicating with the opening formed at the other end of the cylinder 111, and a second hole 123a2 communicating with the first hole 123a1 and the charging part 150.

According to the structure of the first and second holes 123a1, 123a2, in order to communicate the opening formed at the other end of the cylinder 111 with the charging part 150, even without providing a separate hole structure, a flow path through which the compressed air flows between the opening formed at the other end of the cylinder 111 and the charging part 150 may be formed through the first and second holes 123a1, 123a2 by a thickness of the communication part 123a.

Meanwhile, the piston 131 may be provided with a first piston 131a and a second piston 131b.

The first piston 131a faces an opening formed at the other end of the cylinder 111 and is formed to collide with the hammer 113.

The second piston 131b is disposed in the nozzle part 140 to face the first piston 131a. The second piston 131b, as the intake flow path 161 is opened, collides with the first piston 131a that has collided with the hammer 113, and thereby discharges a liquid medicine stored inside the nozzle part 140 to the outside.

On the outer circumferential surfaces of the first piston 131a and the second piston 131b, a first piston seal 131a1 and a second piston seal 131b1 for sealing of the first piston 131a and the second piston 131b may be respectively formed.

Meanwhile, the nozzle part 140 may be provided with a nozzle housing 141 and a piston cushion 142.

The nozzle housing 141 is formed to surround the second piston 131b so that the second piston 131b can be accommodated. The second piston 131b, in a state of being surrounded by the nozzle housing 141, may be movably formed forward and backward.

In addition, the nozzle housing 141 may be provided with an inlet 141a provided at a side portion and a discharge port 141b provided at an end portion through which the liquid medicine is discharged to the outside.

The piston cushion 142 is interposed between the nozzle housing 141 and the second piston 131b, and may provide a resilient force to the second piston 131b so as to restore the second piston 131b moved by collision with the first piston 131a.

In addition, the nozzle part 140 may be further provided with a liquid medicine inlet valve 143 and a liquid medicine discharge valve 144.

In addition, a fluid such as liquid medicine needs to stably move in a single direction from an inlet to an outlet. Further, for blocking or controlling a flow of a fluid in a flowing direction, a check valve is generally used. Such a check valve has been commercialized in various types and can be easily provided, and it is possible to selectively apply it in consideration of a flow direction, pressure, and material of the fluid.

The liquid medicine inlet valve 143 is disposed between the inlet 141a and the inside of the nozzle part 140, and may be formed to selectively open and close the inlet 141a by a pressure difference.

The liquid medicine discharge valve 144 is disposed between the discharge port 141b and the inside of the nozzle part 140, and may be formed to selectively open and close the discharge port 141b by a pressure difference.

Here, the piston 131 may be formed to increase an inside pressure of the nozzle part 140 when colliding with the hammer 113.

A description of a process in which the liquid medicine inlet valve 143 and the liquid medicine discharge valve 144 operate will be given later with reference to other drawings of the present invention.

Meanwhile, a magnetic body 112a having magnetic force may be provided in the casing 112 facing one end of the cylinder 111. Here, the hammer 113 may be formed to be coupled with the magnetic body 112a by a magnetic force. Accordingly, in an operation in which the hammer 113 moves back from the other end of the cylinder 111 to one end of the cylinder 111, after the hammer 113 moves again to an initial position, the hammer 113 may stably maintain a state placed at the initial position.

In addition, on at least one of two surfaces facing each other between the magnetic body 112a and the hammer 113, a hammer cushion 113a formed to absorb impacts when the magnetic body 112a and the hammer 113 collide with each other may be provided.

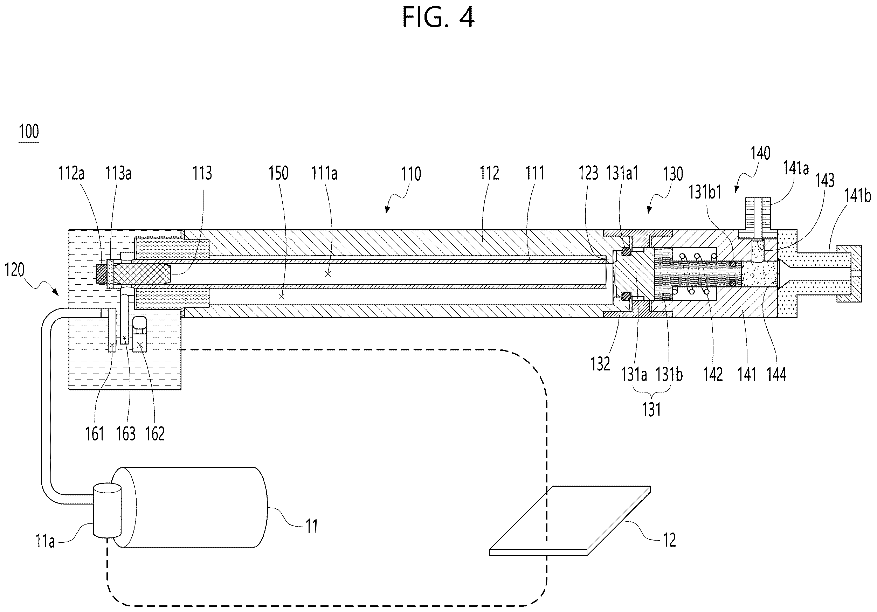

Hereinafter, an operation process of the liquid jet injection device 100 will be described with reference to FIGS. 4 to 8.

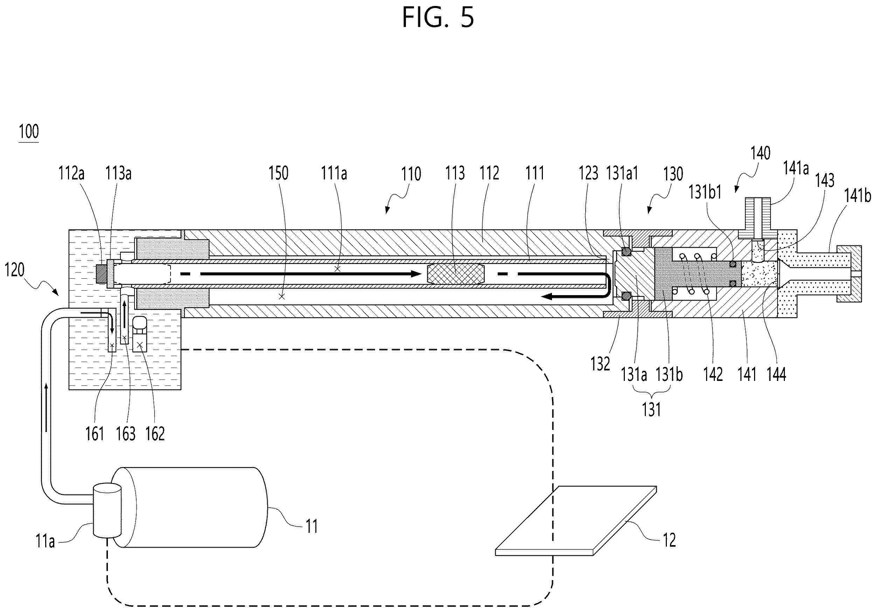

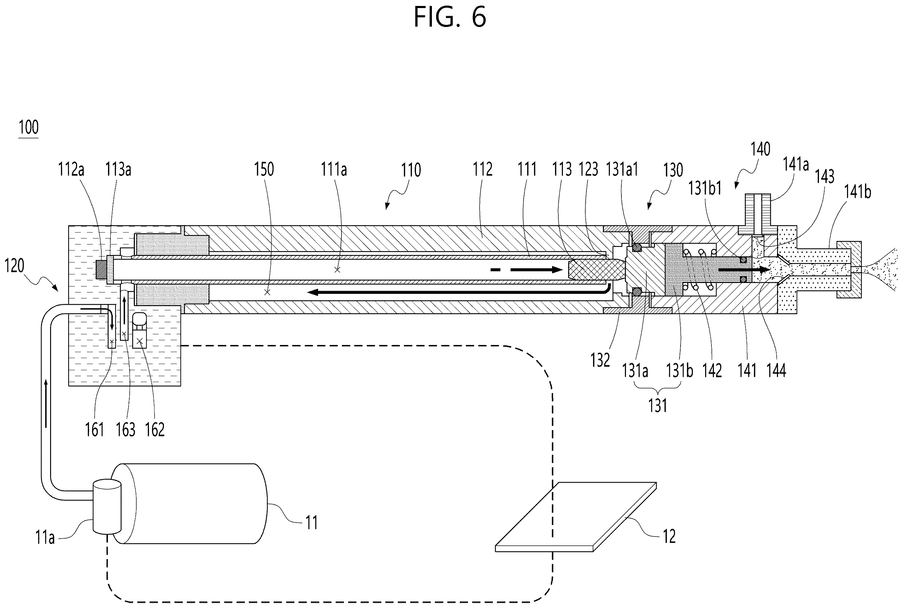

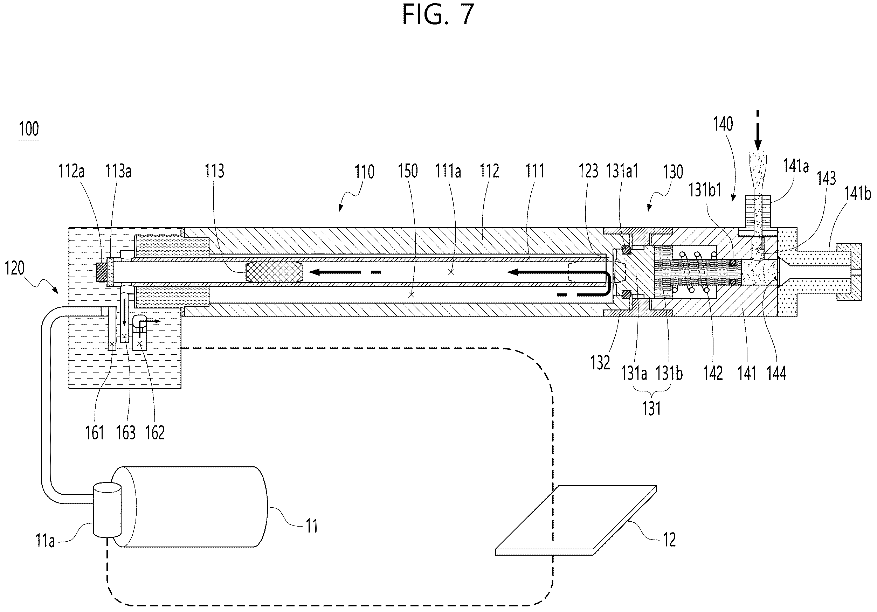

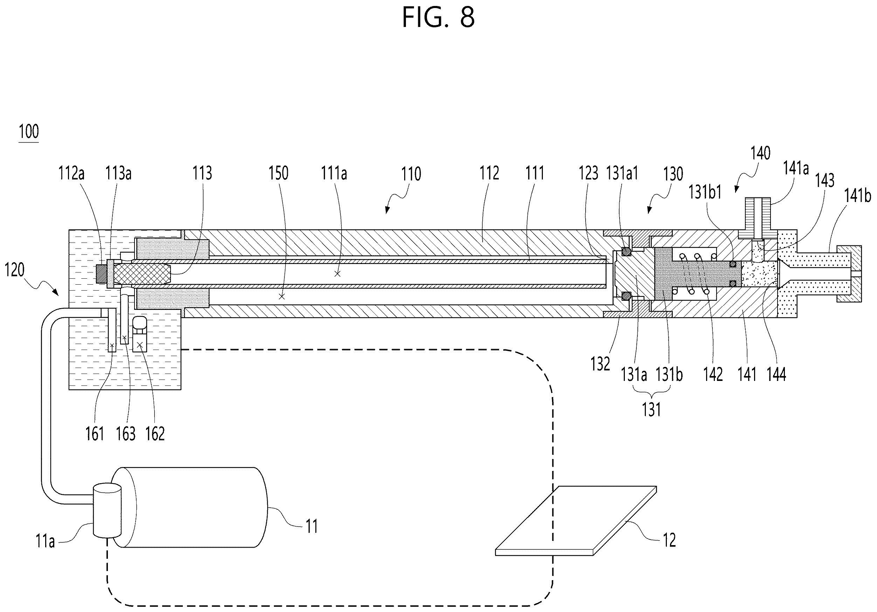

FIG. 4 is a conceptual diagram illustrating a state before the intake flow path 161 of the liquid jet injection device 100 shown in FIG. 1 is opened. FIG. 5 is a conceptual diagram illustrating a state in which, in the liquid jet injection device 100 shown in FIG. 4, the intake flow path 161 is opened, and the hammer 113 moves from one end of the cylinder 111 toward the other end. FIG. 6 is a conceptual diagram illustrating a state in which, in the liquid jet injection device 100 shown in FIG. 5, the hammer 113 moves to the other end of the cylinder 111, collides with the piston 131, and the piston 131 moves a predetermined distance by the impact. FIG. 7 is a conceptual diagram illustrating a state in which, in the liquid jet injection device 100 shown in FIG. 6, after the hammer 113 collides with the piston 131, the exhaust flow path is opened, and the hammer 113 moves toward one end of the cylinder 111. FIG. 8 is a conceptual diagram illustrating a state in which, in the liquid jet injection device 100 shown in FIG. 7, the hammer 113 moves to one end of the cylinder 111 and returns to the initial position.

With reference to FIGS. 4 to 8, compressed air generated from the compressed air-generating part 11 is supplied to the inside 111a of the cylinder 111, and a forward movement of the hammer 113 is generated by an air pressure caused by the compressed air supplied to the inside 111a of the cylinder 111. As described above, the air pressure energy is primarily converted into kinetic energy, the kinetic energy is secondarily converted into collision energy by a collision between the hammer 113 and the first piston 131a, and the collision energy caused by a collision between the first piston 131a and the second piston 131b is again converted into kinetic energy as a third conversion, and the liquid medicine charged inside the nozzle part 140 is pressurized and is configured to be discharged through the discharge port 141b. Subsequently, as the exhaust flow path 162 is opened, by a force of the compressed air compressed and charged in the charging part 150 escaping through the exhaust flow path 162, the hammer 113 returns to the initial position.

More specifically, referring first to FIG. 4, in an initial state of the liquid jet injection device 100, as shown in FIG. 5, when the intake flow path 161 is opened through the control valve 120, the compressed air generated from the compressed air-generating part 11 is supplied to the inside 111a of the cylinder 111 through the intake flow path 161. Further, the compressed air supplied into the inside 111a of the cylinder 111 moves the hammer 113 from one end of the cylinder 111 to the other end of the cylinder 111. In this case, the compressed air is compressed and charged into the charging part 150 formed between the cylinder 111 and the casing 112.

Next, with reference to FIG. 6, the hammer 113 moves to the other end of the cylinder 111 by the compressed air and collides with the first piston 131a, and collision energy of the hammer 113 and the first piston 131a is transmitted to the second piston 131b, thereby pushing the second piston 131b. In this case, the second piston 131b, which has received the collision energy, is converted into high-speed micro motion by the piston cushion 142, and presses and pushes the liquid medicine inside the nozzle part 140 toward the discharge port 141b. Subsequently, as the liquid medicine discharge valve 144 is pressurized and opened, the liquid medicine inside the nozzle part 140 is discharged to the outside through the discharge port 141b.

Next, referring to FIG. 7, after pressurization of the second piston 131b is completed, a negative pressure occurs inside the nozzle part 140, and as the liquid medicine inlet valve 143 provided between the inlet 141a connecting a liquid medicine storage device (not illustrated) of the outside and the inside of the nozzle part 140 is opened, the liquid medicine is charged into the inside of the nozzle part 140 by an applied pressure difference between the inside of the nozzle part 140 and the outside. After charging of the liquid medicine is completed and the negative pressure inside the nozzle part 140 is removed, the liquid medicine inlet valve 143 automatically performs a closing operation. At the same time, as the liquid medicine discharge valve 144 is closed, it is possible to prevent a phenomenon in which the liquid medicine discharged through the discharge port 141b of the nozzle part 140 and blood generated by a wound flow back into the inside.

In addition, as illustrated in FIG. 7, an operation in which the hammer 113 returns to the initial position is performed through a process in which the hammer 113 is pushed by a force of the compressed air compressed and charged in the charging part 150, formed between the cylinder 111 and the casing 112, escaping through the exhaust flow path 162. In addition, an operation in which the hammer 113 returns to the initial position may be partially performed by a collision repulsive force generated after the hammer 113 collides with the first piston 131a.

Finally, as shown in FIG. 8, the hammer 113 returns to the initial position and may stably maintain a state moved to the initial position by the magnetic body 112a disposed at a position facing one end of the cylinder 111.

The foregoing description is merely exemplary, and various modifications may be made by those skilled in the art to which the present invention pertains without departing from a scope and technical teachings of the described embodiments. The described embodiments may be implemented individually or in any combination.

## Claims

1. A liquid jet injection device, comprising:
a cylinder part provided with a cylinder, a casing surrounding the cylinder, and a hammer accommodated in an inside of the cylinder and movably formed between one end and the other end of the cylinder;
a control valve configured to selectively open and close an intake flow path connecting a compressed air-generating part generating compressed air supplied into the inside of the cylinder and one end of the cylinder, and an exhaust flow path connecting one end of the cylinder and the outside;
a pressurizing part provided with a piston facing an opening formed at the other end of the cylinder; and
a nozzle part coupled to the pressurizing part and formed to discharge to the outside a liquid medicine stored therein when the hammer collides with the piston,
wherein a charging part configured to communicate with the other end of the cylinder and in which the compressed air is compressed and charged when the intake flow path is opened, is provided between the casing and the cylinder, and
wherein the hammer, in a state of having moved from one end of the cylinder to the other end of the cylinder by opening of the intake flow path, is configured to move from the other end of the cylinder to one end of the cylinder by the compressed air compressed and charged in the charging part when the exhaust flow path is opened.

2. The liquid jet injection device of claim 1, wherein the hammer, when the intake flow path is opened, moves to the other end of the cylinder by the compressed air supplied to one end of the cylinder and collides with the piston, and
wherein, in a state in which the hammer has moved to the other end of the cylinder, when the exhaust flow path is opened, the hammer is moved to one end of the cylinder by being pushed by an air flow in which the compressed air charged in the charging part escapes to the outside of the cylinder through the exhaust flow path due to an applied pressure difference between the inside of the cylinder and the outside.

3. The liquid jet injection device of claim 1, wherein the charging part is formed to surround a part of a side surface of the cylinder.

4. The liquid jet injection device of claim 3, wherein a partition plate is provided between the other end of the cylinder and the piston, and a communication part formed to communicate an opening formed at the other end of the cylinder with the charging part is provided in the partition plate.

5. The liquid jet injection device of claim 1, wherein a connection flow path communicating with the inside of the cylinder, the intake flow path, and the exhaust flow path is provided between the intake flow path and the exhaust flow path, and
wherein the control valve controls the connection flow path to selectively communicate with any one of the intake flow path and the exhaust flow path.

6. The liquid jet injection device of claim 1, wherein the piston comprises:
a first piston facing an opening formed at the other end of the cylinder and configured to collide with the hammer; and
a second piston disposed in the nozzle part, facing the first piston, and configured to collide with the first piston, which has collided with the hammer as the intake flow path is opened, so as to discharge a liquid medicine stored inside the nozzle part to the outside.

7. The liquid jet injection device of claim 6, wherein the nozzle part comprises:
a nozzle housing configured to accommodate the second piston; and
a piston cushion interposed between the nozzle housing and the second piston, and configured to provide a resilient force to the second piston so as to restore the second piston moved by the collision with the first piston.

8. The liquid jet injection device of claim 1, wherein a magnetic body having a magnetic force is provided in the casing facing one end of the cylinder, and the hammer is formed to be coupled with the magnetic body by the magnetic force.

9. The liquid jet injection device of claim 8, wherein a hammer cushion formed to absorb impacts when the magnetic body and the hammer collide with each other is provided on at least one of two surfaces facing each other between the magnetic body and the hammer.

10. A liquid jet injection device, comprising:
a cylinder part provided with a cylinder, a casing surrounding the cylinder, and a hammer accommodated in an inside of the cylinder and movably formed between one end and the other end of the cylinder;
a control valve configured to selectively open and close an intake flow path connecting a compressed air-generating part generating compressed air supplied into the inside of the cylinder and one end of the cylinder, and an exhaust flow path connecting one end of the cylinder and the outside;
a pressurizing part provided with a piston facing an opening formed at the other end of the cylinder; and
a nozzle part coupled to the pressurizing part and formed to discharge to the outside a liquid medicine stored therein when the hammer collides with the piston,
wherein the hammer, when the exhaust flow path is opened, is configured to be restored from the other end of the cylinder to one end of the cylinder by the compressed air, which is caused to be compressed inside the casing when the intake flow path is opened.
